# EUROPÄISCHE PATENTANMELDUNG

(11) **EP 2 979 660 A1**
(43) Veröffentlichungstag der Anmeldung: **03.02.2016**
(21) Anmeldenummer: 14472002.6
(22) Anmeldetag: 28.07.2014
(51) Int. Cl.: A61B 18/14

(54) **System für HF-Ablation**

(71) Anmelder: Amet Ood, 1331 Sofia (BG)
(72) Erfinder: Popov, Theodor Stefanov, Sofia (BG)

(57) **Zusammenfassung**

Die Erfindung ist in der Medizin für wärmeinduzierte Tötung von Tumorbildungen anwendbar.

Sie löst das Problem der Hochfrequenzeinwirkung ohne Stör - Leckströme außerhalb der Ablationszone und ohne zusätzliche Wärmeverletzungen von Haut und Biogewebe, indem sie eine Behandlung auch von Patienten mit elektronischen Implanten bei erleichterter Bedienung gewährleistet.

Gemäß der Erfindung wird der Bipolarbetrieb der Hochfrequenz - Thermoablation von Tumoren mit Aplikator 11 - Bipolarelektrode verwendet, jeder der Polen welches mit dem regelbaren Speisegenerator 2, mit dem Folgeblock 7 der in das behandelten Gewebe abgegebenen Strom und Spannung und mit dem Kanal 10 für Infusionslösung der Infusionspumpe 5 verbunden ist. Der regelbare Speisegenerator 2 besitzt einen eingebauten zusätzlichen Regelblock 12 für telegraphische Manipulation des Ausgangssignals, verbunden mit Kontroller 1.

Die Patentansprüche sind im Punkt 1 formuliert.

## Beschreibung

### Technikbereich

Die Erfindung betrifft das System für Hochfrequenzablation, die eine Anwendung in der Medizin für wärmeinduzierte Tötung von Tumorbildungen findet.

### Vorgangszustand der Technik

Es ist ein System für hohfrequenz - Thermoablation [1] bekannt, bei welchem die lokale Behandlung der Tumorbildungen im monopolaren Betrieb, mit/ohne Einspritzen von physiologischer Lösung geschieht. Es besteht aus Kontroller, in zwei Richtungen mit steuerbarem Hochfrequenz - Speisegenerator verbunden, mit Interface für Außenangabe der Betriebsparameter (Ausgangsleistung, Applikationszeit und Einflussform) und mit einem Block für Kommunikation mit Außeneinrichtungen. Der letzte ist in zwei Richtungen mit einer Infusionspumpe mit kontrollierter Fördermenge verbunden. Zwei Eingänge des Kontrollers sind mit Ausgängen von zwei Blöcken für Rückverbindung verbunden: mit einem Block für Verfolgung der momentanen Gewebetemperatur in der Ablationszone und mit einem Folgeblock für die in das behandelten Gewebe abgegebenen Strom und Spannung. Ein anderer Ausgang des Kontrollers ist mit dem Block Datenbasis für laufend angewendeten Leistung, Gewebeimpedanz und Temperatur verbunden. Aus dem Block für Verfolgung der momentanen Gewebetemperatur ist eine flexible Thermosonde, positioniert in dem Tumorgewebe ausgeführt. Die Infusionspumpe mit kontrollierter Fördermenge besitzt einen Kanal für Infusionslösung, verbunden mit Applikator - monopolarer Nadelelktrode für Behandeln des Tumorgewebes, der mit dem regelbaren Speisegenerator und mit dem Folgeblock für die in das behandelten Gewebe abgegebenen Strom und Spannung verbunden ist. Von der äußeren Patientenseite ist dicht zur Haut eine Neutralelektrode positioniert die auch mit dem regelbaren Speisegenerator und mit dem Folgeblock für die in das behandelten Gewebe abgegebenen Strom und Spannung verbunden ist.

Dieses bekanntes System mit Monopolarbetrieb der Thermoablation führt zu bemerkbaren Nebeneffekten: Als Grundunbequemlichkeit der Monopolarablation ist die Notwendigkeit von einer oberflächlichen Außen - Neutralelektrode und das Schließen der Kraftlinien der HF - Stromfeldes durch de n Applikator über die Außen - Neutralelektrode führt zur Abgabe von schädlicher Energie in das Volumen der Biomasse, geschlossen zwischen den beiden Elektroden. So geschieht eine unerwünschte Abgabe von Mengen Wärmeenergie nicht nur in den Tumor, sondern auch in das umschliessende gesunde Gewebe in den Zonen mit hoher Felddichte und das Ergebnis, eine solche Erwärmung nur um die Spitze des Applikators - der aktiven Elektrode, die in dem geometrischen Zentrum der Tumorbildung gelegt ist, zu bestehen, wird nicht erreicht. Komplizierter ist auch die Bedienung des bekannten Systems - bei schlecht realisierter dichter elektrischer Verbindung zwischen der Außen - Neutralelektrode und der Patientenhaut entstehen Zonen von schädlicher hoher Dichte der Kraftlinien unter der Neutralelektrode und oberflächliche Verbrennungen der Epidermisschicht.

Eine andere Unbequemlichkeit ist der begrenzte Zugang von Patienten - eine Behandlung von Patienten mit elektronischen Implanten (z.B. Pacemaker) ist wegen Risiko von Störung ihres Betriebes durch die Hochfreqenzenergie, die durch den Körper fließt unzulässig - dieses Problem basiert auf die gegenseitige räumliche Anordnung zwischen den Elektroden und dem Implanten, auf die mehreren Parameter des lebendigen Organismus und auf die Menge der abgegebenen Leistung; deswegen gilt die monopolare Thermoablation als bei Patienten mit einem ähnlichem Status nicht indiziert.
Die beschriebenen Probleme und Risiken werden durch die vorliegende Erfindung, die den Bipolarbetrieb der Thermoablation verwendet, überwunden.

### Technisches Wesen der Erfindung

Die Aufgabe der Erfindung ist, ein System für Hochfrequenzablation zu entwickeln, das einen Einfluss der Tumorbildungen ohne Stör - Leckströme außerhalb der Ablationszone und ohne zusätzliche Wärmeverletzungen von Haut und Biogewebe außerhalb der behandelten Zone zu schaffen und eine Behandlung auch von Patienten mit elektronischen Implanten bei erleichterter Bedienung zu gewährleisten.

Die Aufgabe ist mit der Erfindung gelöst, indem ein System für Hochfrequenz - Thermoablation entwickelt ist, bei welchem die lokale Behandlung der Tumorbildungen bipolar ist. Es besteht aus Kontroller, in zwei Richtungen mit einem regelbaren HF-Speisegenerator verbunden, mit Interface für Ferneingabe der Betriebsparameter (Ausgangsleistung, Applikationszeit und Einwirkungsform) und mit einem Block für Kommunikation mit Außeneinrichtungen. Der letzte ist mit einer Infusionspumpe mit kontrollierter Fördermenge verbunden. Zwei Eingange des Kontrollers sind mit Ausgängen der zwei Blöcke für Rückverbindung verbunden: mit einem Block für Verfolgung der momentanen Gewebetemperatur in der Ablationszone und mit einem Folgeblock für die in das behandelten Gewebe abgegebenen Strom und Spannung. Ein anderer Ausgang des Kontrollers ist mit einem Block - Datenbasis für die laufend verwendeten Leistung, Gewebeimpedanzen und Temperatur. Aus dem Block für Verfolgung der momentanen Gewebetemperatur ist eine flexible Thermosonde, positioniert in dem Tumorgewebe ausgeführt. Die Infusionspumpe mit kontrollierter Fördermenge besitzt einen Kanal für Infusionslösung, verbunden mit jedem der Polen des Applikators - Bipolarelektrode für Behandeln des Tumorgewebes, wobei jede der Elektroden auch mit dem regelbaren Speisegenerator und mit dem Folgeblock für die in das behandelten Gewebe abgegebenen Strom und Spannung verbunden ist. Der regelbare Speisegenerator besitzt einen zusätzlichen Block für telegraphische Manipulation des Ausgangssignals, verbunden mit dem Kontroller.

Die Vorteile der Erfindung sind Folge der Schemenmodifikation - Einfügen einer zweiten Stufe der Freiheit bei der Steuerung des Hochfrequenzgenerators, was eine Feineinstellung der in das Gewebe abgeführten Leistung erlaubt und mit dem Softwarealgorithmus erhöht man die Sicherheit und de Effektivität der Therapie, bei Vermeiden der Gewebekarbonisierung.

Die Bipolarapplikatoren kombinieren in sich sowohl die aktive, als auch die Neutralelektrode. Da sie eine gleiche Fläche haben, entsteht um sie eine effektive Zone mit erhöhter Dichte der Kraftstromlinien - insgesamt zwei Zonen (bei der monopolaren Ablation besteht nur eine solche Zone). Die beiden Elektroden sind im Tumor angeordnet und das bestimmt auch die abgegebene in Volumeneinheit Menge Wärmeenergie - bei der bipolaren Ablation ist diese Menge grösser und konzentrierter im Vergleich zu der monopolaren Ablation. Das gewährleistet ein kleineres Risiko von kolateralen Verletzungen in dem abgrenzenden gesunden Gewebe.

Durch die Bipolarablation wird eine grössere Tiefe der Durchdringung und eine stark lokalisierte Einwirkung auf die Tumorbildung ohne Stör - Leckströme gewährleistet. Die Verteilung der Kraftstromlinien und die Verbreitung der Wärmemenge im Gewebe zeichnen sich durch wesentlich höhere Dichte und Gradient aus, was zu einer starken lokalen Erwärmung von kleinen Tumorvolumen und einen effektiven Betrieb bei niedrigeren Spannungen führt.

Weitere Vorteile des Systems mit HF - bipolarer Thermoablation sind auch die erleichterte Bedienung und die bessere Sicherheit der Patienten, es wird auch die Menge der erforderlichen Konsumativen gemindert - es werden nicht so viel äußere Einweg - Neutralelektroden gekauft und aufbewahrt, entfällt auch das Risiko bei der falschen Einlegung der Elektroden und Verschiebung bei unvorhergesehener und unerwarteter momentaner Bewegung des Patientenkörpers.

Die Erfindung erreicht auch **zusätzliche Vorteile** (*außerhalb der Aufgabe*) aus der Art und Weise der Lösung: Für Behandeln von Tumoren mit einem grösseren Volumen (über 500 mm Durchmesser bei annähernder Kugelform der Bildung) ist eine Perfusion der mediatorischen Flüssigkeit in der Ablationszone erforderlich, indem gleichzeitig zusätzlich noch zwei nützliche Effekte erreicht werden: die Kühlung der Bipolarnadel, was die Karbonisierung des Gewebes um die Elektroden verhindert und die Kühlung des behandelten Gewebes, was die Senkung des Temperaturgradienten und eine Tiefbehandlung bis zu den entfernten von den Elektroden Zonen erlaubt. Es besteht noch ein neuer dritter Effekt: Aufrechterhalten der ständigen elektrischen Eigenschaften des Mediums zwischen den Elektroden, welcher Effekt besonders wichtig wegen der (selbst)Regulierung der Parameter des Einflusses bei der Bipolarablation, bei charakterlichen für sie starken Änderungen der Gewebeimpedanz ist.

### Erklärung der beigelegten Figuren

Ausführlicher wird die Erfindung mit den beigelegten Figuren erklärt, wo:
Figur 1 das Funktionsschema des Systems gemäß der Erfindung darstellt;
Figur 2 das Algorithmus der ersten Etappe der Ablationseinwirkung gemäß der Erfindung darstellt;
Figur 3 der Algorithmus der zweiten Etappe der Ablationseinwirkung gemäß der Erfindung darstellt.

### Beispiele für Ausführung der Erfindung

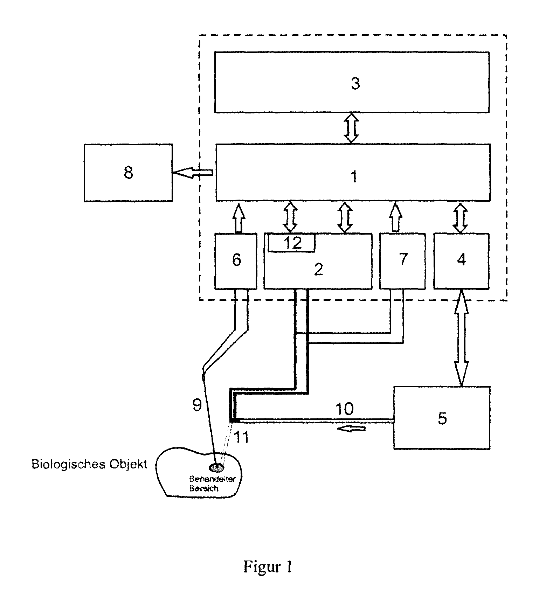

An der beigelegten Figur 1 ist ein System für Hochfrequenz - Thermoablation von Tumoren in dem Bauchbereich dargestellt, bei welchem die lokale Behandlung der Tumorbildungen bipolar ist. Es besteht aus Kontroller 1, in zwei Richtungen mit einem regelbaren HF Speisegenerator 2 verbunden, mit Interface 3 für Ferneingabe der Betriebsparameter (Ausgangsleistung, Applikationszeit und Einwirkungsform) und mit Block 4 für Kommunikation mit Außen - Einrichtungen, der mit der Infusionspumpe 5 mit kontrollierter Fördermenge und mit einem PC für Datenbasis als solche Einrichtungen verbunden ist. Zwei Eingänge des Kontrollers 1 sind mit Ausgängen der zwei Blöcke für Rückverbindung verbunden: mit einem Block 6 für Verfolgung der momentanen Gewebetemperatur in der Ablationszone und mit einem Folgeblock 7 für die in das behandelten Gewebe abgegebenen Strom und Spannung, die auch mit der Datenbasis eines PCs verbunden werden können.. Ein anderer Ausgang des Kontrollers 1 ist mit einem Block 8 - Datenbasis für die laufend verwendeten Leistung, Gewebeimpedanzen und Temperatur. Aus dem Block für Verfolgung der momentanen Gewebetemperatur 6 ist eine flexible Thermosonde 9, z.B. Thermopaar (K-Typ), positioniert in dem Tumorgewebe ausgeführt. Die Infusionspumpe mit kontrollierter Fördermenge 5 besitzt einem kanal für Infusionslösung 10 , verbunden mit jedem der Polen des Applikators 11 - Bipolarelektrode für Behandeln des Tumorgewebes, wobei jede der Elektroden auch mit dem regelbaren Speisegenerator 2 und mit dem Folgeblock 7 für die in das behandelten Gewebe abgegebenen Strom und Spannung verbunden ist. Der regelbare Speisegenerator 2 besitzt einen zusätzlichen Block 12 für telegraphische Manipulation des Ausgangssignals, verbunden mit dem Kontroller 1.

Die Ausführung des Applikators 11 als Bipolarelektrode führt zu keinen Komplikationen des Systems, da schon entwickelte Konstruktionen solcher Elektroden bestehen, darunter solcher, mit sich verlängerten Segmenten, flexibler Dilatatoren der aktiven Fläche u.a. für Erhöhung der Effektivität der Therapie und die Parameter der Applikatorennadel können in breiten Grenzen varieren und erlauben eine Optimierung.

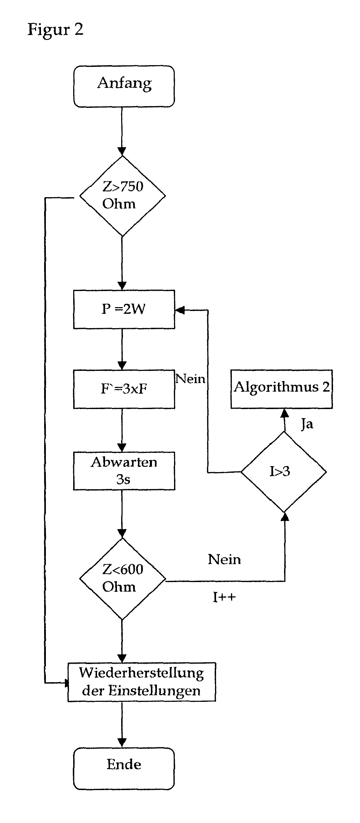

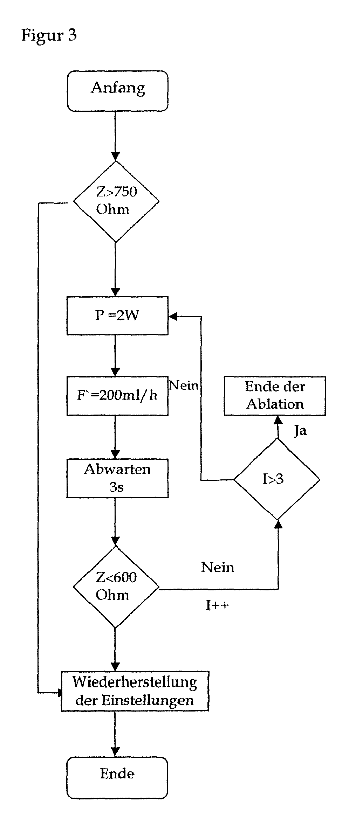

Die Wirkung des Systems für Hochfrequenz - Thermoablation gemäß der Erfindung basiert auf ein Algorithmus für Prevenz der Gewebekarbonisierung durch Bipolarablation, der in zwei Ebenen in Abhängigkeit von dem Grad der Einwirkung auf dem Tumor ausgeführt wird - primäre und volle Variante. Sie sind in Fig. 2 und Fig. 3 dargestellt und werden nacheinander ausgeführt; in der ersten Ebene wird immer der primäre Algorithmus der Fig. 2 gestartet. Wenn die für ihn vorgeschriebenen Handlungen das Erfolgskriterium nicht erfüllen, wird die volle Variante der Fig. 3 als zweite Ebene aktiviert.
Durch die Neueinführung wird die telegraphische Manipulation des HF - Ausgangssignals aus dem Generator aktiviert, indem der effektive Wert der abgegebenen Leistung geändert wird und in derselben Zeit die hohen Amplituden des Ausgangsstroms und der Ausgangsspannung erhalten bleiben. Das erlaubt eine große Tiefe der Durchdringung der Hochfrequenzenergie in das Gewebe (Dank der hohen effektiven Werte der für den Generator Ausgangs - Stromparameter) bei Erwärmung mit reduziertem Temperaturgradient (kontrolliert von der Signalmanipulierung). Die beiden Elektroden des Bipolarapplikators 11 - sowohl die aktive, als auch die neutrale Elektrode werden im Tumor angeordnet und bilden **zwei effektive Zonen** mit erhöhter Dichte der Kraftstromlinien des generierten elektrischen Feldes, geschlossen zwischen den beiden nahestehenden Elektroden. Die so abgegebene Energiemenge in Volumeneinheit ist bei der Bipolarablation erhöht und konzentrierter im Vergleich zu der monopolaren Ablation, indem das Risiko von kolateralen Verletzungen in dem abgrenzenden gesunden Gewebe eliminiert und die Geschwindigkeit der Gewebekarbonisierung reduziert wird. Gleichzeitig damit werden auch das effektive Ablationsvolumen und die therapeutische Durchdringungsfähigkeit erhöht, ohne damit qualitätsmäßig das Verhalten des Gewebes geändert zu werden, indem die Therapiezeit für Erreichen einer gleichmäßigen Erwärmung des Zweckvolumens erhöht wird. Zum Kontroller 1 wird eine Information aus den Blöcken für Rückverbindung-Block 6 für Verfolgen der Gewebetemperatur in dem behandelten Berich und Block 7 für Messen der abgegebenen Leistung und Gewebeimpedanz, gegeben. Die Werte dieser Parameter können auch zur Datenbasis eines PCs gesendet werden. Die Steuerung des Blocks für telegraphische Manipulation 12 geschieht von dem Kontroller 1. Bei der Verwendung von physiologischer Lösung als Kühleffekt und Reduzierung der Impedanz wird der Wert der Fördermenge automatisch nach der Messung mittels Folgeblock 7 der abgegebenen in das behandelten Gewebe Strom und Spannung geregelt. Bei Aktivieren der vollen Variante des Algorithmus der Fig. 3 wird die Fördermenge der physiologischen Lösung automatisch auf Höchstwert eingestellt, indem außer Kühlung und Reduzierung der Impedanz auch eine Reinigung der Perfusionsöffnungen des Applikators 11 vorgenommen wird.
[1] Y. Ni, S. Mulier, Y. Miao, L. Michel, G. Marchal, A review of the general aspects of radiofrequency ablation, Abdominal Imaging (2005) 30:381-400, DOI: 10.1007/s00262-004-0253-9, Springer Science+Business Media, Inc. 2005

## Patentansprüche

1. System für Hohfrequenz - Thermoablation, bestehend aus Kontroller, in zwei Richtungen mit steuerbarem Hochfrequenz - Speisegenerator verbunden, mit Interface für Außenangabe der Betriebsparameter (Ausgangsleistung, Applikationszeit und Einflussform) und mit einem Block für Kommunikation mit Außeneinrichtungen, wobei der letzte in zwei Richtungen mit einer Infusionspumpe mit kontrollierter Fördermenge verbunden ist, zwei Eingänge des Kontrollers sind mit Ausgängen von zwei Blöcken für Rückverbindung verbunden: mit einem Block für Verfolgung der momentanen Gewebetemperatur in der Ablationszone und mit einem Folgeblock für die in das behandelten Gewebe abgegebenen Strom und Spannung, ein anderer Ausgang des Kontrollers ist mit dem Block Datenbasis für laufend angewendeten Leistung, Gewebeimpedanz und Temperatur verbunden, aus dem Block für Verfolgung der momentanen Gewebetemperatur ist eine flexible Thermosonde, positioniert in dem Tumorgewebe ausgeführt, das System besitzt eine Infusionspumpe mit kontrollierter Fördermenge, deren Kanal für Infusionslösung mit der aktiven Elektrode des Applikators, mit dem regelbaren Speisegenerator und mit dem Folgeblock für die in das behandelten Gewebe abgegebenen Strom und Spannung verbunden ist.
***sich damit charakterisierend**, **dass***
der regelbare HF - Speisegenerator (2) einen zusätzlichen Regelblock für telegraphische Manipulation des Ausgangssignals (12) besitzt, verbunden in zwei Richtungen mit dem Kontroller (1), der Applikator (11) ist als Bipolarelektrode ausgeführt, deren zweite Neutralelektrode auch mit dem regelbaren HF - Speisegenerator (2) und mit dem Folgeblock (7) für Strom und Spannung, abgegeben in das behandelten Gewebe und mit dem Kanal für die Infusionslösung (10) der Infusionspumpe (5) verbunden ist.
